(19) Europäisches Patentamt
European Patent Office
Office européen des brevets

(11) **EP 4 304 548 B1**

(12) **EUROPEAN PATENT SPECIFICATION**

(45) Date of publication and mention
of the grant of the patent:
**23.07.2025 Bulletin 2025/30**

(21) Application number: **22713220.6**

(22) Date of filing: **10.03.2022**

(51) International Patent Classification (IPC):
*A61K 8/81* *(2006.01)*      *A61K 8/19* *(2006.01)*
*A61K 8/26* *(2006.01)*      *A61K 8/9789* *(2017.01)*
*A61Q 15/00* *(2006.01)*

(52) Cooperative Patent Classification (CPC):
**A61K 8/26; A61K 8/19; A61K 8/8147;**
**A61K 8/9789; A61Q 15/00**

(86) International application number:
**PCT/US2022/019827**

(87) International publication number:
**WO 2022/192587 (15.09.2022 Gazette 2022/37)**

(54) **ANTIPERSPIRANT COMPOSITION**

ANTITRANSPIRANT ZUSAMMENSETZUNG

COMPOSITION ANTIPERSPIRANTE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **12.03.2021 US 202163160343 P**

(43) Date of publication of application:
**17.01.2024 Bulletin 2024/03**

(73) Proprietor: **Coty Inc.**
**New York, NY 10018 (US)**

(72) Inventors:
• **TEIXEIRA, Jose Elvecio dos Santos**
**06657-460 São Paulo (BR)**
• **ARAKI, Diego Takeshi Higa**
**09861-310 São Paulo (BR)**
• **LORENZETTI, Danielle Lima**
**6454-080 São Paulo (BR)**

(74) Representative: **Dilg, Haeusler, Schindelmann**
**Patentanwaltsgesellschaft mbH**
**Leonrodstraße 58**
**80636 München (DE)**

(56) References cited:
**WO-A1-2017/070115**

• **HAPPI: "Balance Oily Skin With Nelupure", 27 October 2017 (2017-10-27), XP002806941, Retrieved from the Internet <URL:https://www. happi.com/contents/view_breaking-news/ 2017-10-27/balance-oily-skin-with-nelupure/> [retrieved on 20220621]**
• **DATABASE GNPD [online] MINTEL; 19 August 2020 (2020-08-19), ANONYMOUS: "24h Anti-Perspirant Stick", XP055934202, retrieved from https://www.gnpd.com/sinatra/recordpage/ 8036779/ Database accession no. 8036779**
• **DATABASE GNPD [online] MINTEL; 24 June 2015 (2015-06-24), ANONYMOUS: "Qi Gong 24h Anti-Perspirant Spray", XP055934213, retrieved from https://www.gnpd.com/sinatra/recordpage/ 3269099/ Database accession no. 3269099**

**EP 4 304 548 B1**

**Description**

**BACKGROUND**

Consumers desire antiperspirant and deodorant compositions that provide a desired and long-lasting fragrance or scent each time the composition is applied or used. Particularly in the case of deodorants, consumers may also expect compositions that provide a scent that can mask or override other undesirable odors. WO2017/070115A1 describes an antiperspirant composition.

**SUMMARY OF THE DISCLOSURE**

The present disclosure provides an antiperspirant composition that includes active component and an inactive component. The antiperspirant composition can be present as a stick antiperspirant, a body spray, a clear gel, a roll on, a cream, or an aerosol antiperspirant. The active component can include aluminum salt, magnesium salt, film former, extract solution, or a mixture thereof. The extract solution can include nymphaea coerulea flower extract and nelumbo nucifera flower extract.

**DETAILED DESCRIPTION**

Reference will now be made in detail to certain embodiments of the disclosed subject matter.

Throughout this document, values expressed in a range format should be interpreted in a flexible manner to include not only the numerical values explicitly recited as the limits of the range, but also to include all the individual numerical values or sub-ranges encompassed within that range as if each numerical value and sub-range is explicitly recited. For example, a range of "about 0.1% to about 5%" or "about 0.1% to 5%" should be interpreted to include not just about 0.1% to about 5%, but also the individual values (e.g., 1%, 2%, 3%, and 4%) and the sub-ranges (e.g., 0.1% to 0.5%, 1.1% to 2.2%, 3.3% to 4.4%) within the indicated range. The statement "about X to Y" has the same meaning as "about X to about Y," unless indicated otherwise. Likewise, the statement "about X, Y, or about Z" has the same meaning as "about X, about Y, or about Z," unless indicated otherwise.

In this document, the terms "a," "an," or "the" are used to include one or more than one unless the context clearly dictates otherwise. The term "or" is used to refer to a nonexclusive "or" unless otherwise indicated. The statement "at least one of A and B" has the same meaning as "A, B, or A and B." In addition, it is to be understood that the phraseology or terminology employed herein, and not otherwise defined, is for the purpose of description only and not of limitation. Any use of section headings is intended to aid reading of the document and is not to be interpreted as limiting; information that is relevant to a section heading may occur within or outside of that particular section.

According to various aspects of the present disclosure an antiperspirant composition can include an active component and an inactive component. The antiperspirant composition can be present as a stick antiperspirant, a body spray, a clear gel, a roll on, a cream, or an aerosol antiperspirant. The active component can include an aluminum salt or magnesium salt, a film former, and an extract solution. The extract solution can include nymphaea coerulea flower extract and nelumbo nucifera flower extract. It has been surprisingly and unexpectedly found that the combination the aluminum salt, magnesium salt, film former, extract solution, or a mixture thereof can produce an antiperspirant composition that is capable of reducing perspiration by 40% to 70%, or from 55% to 65%, less than, equal to, or greater than about 40%, 41, 42, 43, 44, 45, 46, 47, 48, 49, 50, 51, 52, 53, 54, 55, 56, 57, 58, 59, 60, 61, 62, 63, 64, 65, 66, 67, 68, 69, or about 70% over a time in a range of from about 40 hours to 100 hours, or from 90 hours to 100 hours, less than, equal to, or greater than about 40 hours, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or about 100 hours-relative to an untreated portion of skin or an antiperspirant composition that is free of any of the three components. Without intending to be bound by any theory, it is suspected that the increased performance of the instant antiperspirant composition can be at least partially attributed to the role of the extract solution acting on preventing perspiration that is produced and not able to be blocked or absorbed by the aluminum salt or magnesium salt. Aluminum salts have a maximum amount of perspiration that they are able to address and the extract solution is thought to address the excess amount. The film former is thought to be able to facilitate substantial homogenation of the extract solution and aluminum salt and evenly distribute the solution about a user's skin.

The extract solution can be NELUPURE, available from Centerchem. NELUPURE includes propanediol, glycerin, nymphaea caerulea flower extract, and nelumbo nucifera flower extract.

The aluminum salt or magnesium salt can constitute any suitable amount of the antiperspirant composition can be in a range of from about 1 wt% to about 55 wt%, 25 wt% to 55 wt,%, about 1 wt% to about 10 wt% of the active component, about 35 wt% to about 45 wt% of the active component, less than, equal to, or greater than about 25 wt%, 26, 27, 28, 29, 30, 31, 32, 33, 34, 35, 36, 37, 38, 39, 40, 41, 42, 43, 44, 45, 46, 47, 48, 49, 50, 51, 52, 53, 54, or about 55 wt%. Antiperspirant salts useful as antiperspirant salts or as components of antiperspirant complexes include aluminum halides, aluminum hydroxy-halides, zirconyl oxyhalides, zirconyl hydroxy-halides, and mixtures of these materials. A specific aluminum salt can include aluminum sesquichlorohydrate.

Examples of magnesium salts include magnesium carbonate, magnesium carbonate, magnesium oxide, magnesium hydroxide, magnesium citrate, magnesium chloride, or a mixture thereof. Where the magnesium salt is used, a ratio of the magnesium salt to the NELUPURE can be in a range of from about 0.5:8, 1:2, 1:5, or 2:8.

Aluminum salts of this type include aluminum chloride and the aluminum hydroxyhalides having the general formula $Al_2(OH)xQyXH_2O$ where Q is chlorine, bromine or iodine; where x is from about 2 to about 5, and x+y=about 6, and x and y do not need to be integers; and where X is from about 1 to about 6. Aluminum salts of mis type can be prepared in the manner described more fully in U.S. Pat. No. 3.887.692 issued to Gilman on Jun. 3, 1975, and U.S. Pat. No. 3.904.741 issued to Jones and Rubino on Sep. 9, 1975.

The zirconium compounds which are useful in the present invention include both the zirconium oxy salts and zirconium hydroxyl salts, also referred to as the zirconyl salts and zirconyl hydroxy salts. These antiperspirant actives are further described in US 7897799 Specific antiperspirant active salts usable in the formula embodiment include one or more of the following: aluminum zirconium tetrachlorhydrex glycine complex with zinc glycinate and aluminum zirconium tetrachlorhydrex glycine complex with a salt other than zinc glycinate such as sodium glycinate and other water soluble amino acid salts such as sodium alginate. Other active solid antiperspirants include aluminum chlorhydrate, aluminum sesquichlorohydrate, aluminum zirconium trichlorohydrex glycine, aluminum zirconium pentachlorohydrex glycine, aluminum zirconium tetrachlorohydrex glycine and aluminum zirconium octochlorohydrex glycine. The aluminum zirconium-containing materials are commonly referred to as antiperspirant active aluminum zirconium salts. Generally, the foregoing metal antiperspirant active materials are antiperspirant active metal salts listed in the Federal Register, Vol. 68, No. 110/Monday, Jun. 9, 2003/Rules and Regulations.

Salts useful as antiperspirant salts or as components or complexes include aluminum halides, aluminum hydroxy-halides, zirconyl oxyhalides, zirconyl hydroxy-halides, and mixtures of these materials.

The film former can be in a range of from about 1 wt% to 20 wt%, 2 wt% to about 20 wt% of the active component, about 7 wt% to about 13 wt% of the active component, less than, equal to, or greater than about 2 wt%, 3, 4, 5, 6, 7, 8, 9, 10, 11, 12, 13, 14, 15, 16, 17, 18, 19, or about 20 wt%. As described herein above, the film former can be used to help produce a homogenous mixture of the aluminum salt and the extract solution as well as help to evenly distribute the antiperspirant composition about a user's skin. According to various aspects, the film former includes Sodium Polyacrylate

The extract can be in a range of from about 0.10 wt% to about 5 wt% of the active component, about 0.5 wt% to about 1.5 wt% of the active component, less than, equal to, or greater than about 0.5 wt%, 0.6, 0.7, 0.8, 0.9, 1.0, 1.1, 1.2, 1.3, 1.4, or about 1.5 wt%. In addition to the nymphaea coerulea flower extract and nelumbo nucifera flower extract, the extract solution can include additional components. Those additional components can include propanediol and glycerin. In some examples, the nymphaea coerulea flower extract is in a range of from about 0.00075 wt% to about 0.04 wt%, 0.001 wt% to about 0.02 wt% of the extract, 0.001 wt% to about 0.01 wt% of the extract, about 0.005 wt% to about 0.009 wt%, less than equal to, or greater than about 0.001 wt%, 0.005, 0.009, or 0.01 wt%. The nelumbo nucifera flower extract can be in a range of from about 0.00075 wt% to 0.04 wt%, about 0.001 wt% to 0.02 wt% of the extract, 0.001 wt% to about 0.01 wt% of the extract, about 0.005 wt% to about 0.009 wt%, less than equal to, or greater than about 0.001 wt%, 0.005, 0.009, or 0.01 wt%. The propanediol can be in a range of from about 0.08 wt% to about 4 wt%, about 0.1 to 1.6 wt%, 0.5 to about 1.5 wt% of the extract, about 0.6 wt% to about 1 wt%, less than, equal to, or greater than about 0.5 wt%, 0.6, 0.7, 0.8, 0.9, 1.0, 1.1, 1.2, 1.3, 1.4, or about 1.5 wt%. The glycerin can be in a range of from about 0.01 wt% to about 1 wt%, about 0.03 wt% to 0.4 wt% of the extract, 0.05 wt% to about 0.2 wt% of the extract, about 0.07 wt% to about 0.1 wt%, less than, equal to, or greater than about 0.05 wt%, 0.06, 0.07, 0.08, 0.09, 0.1, or about 0.2 wt%.

The antiperspirant composition can include a solvent. The solvent can be an organic solvent. Where present, the solvent is in a range of from about 1 wt% to 50 wt% or 10 wt% to about 50 wt% of the active component, about 30 wt% to about 40 wt% of the active component, less than, equal to, or greater than about 30 wt%, 31, 32, 33, 34, 35, 36, 37, 38, 39, 40, 41, 42, 43, 44, 45, 46, 47, 48, 49, or about 50 wt% of the antiperspirant composition. Examples of organic solvents can include an isododecane component, an alkyl benzoate component, and a neopentyl glycol diheptanoate component. Isododecane (an example of which is represented by CAS 31807-55-3) is generally understood to be a clear, colorless and odorless, volatile liquid, which makes it suitable for use in color cosmetics like mascara, eyeliner, lip products, antiperspirant or any product where improved wear properties and no residues are wanted. It does not leave an oily residue. Isododecane is a volatile, lipophilic component for deodorant sprays and hair care applications. It is a hydrocarbon ingredient used as a solvent. The alkyl benzoate component can be C12-15 alkyl benzoate (an example of which is represented by CAS 68411-27-8), which is generally understood to be a low-molecular weight ester of benzoic acid and C12-15 alcohols. C12-15 alkyl benzoate is a clear liquid that is practically odorless. In some examples, the alkyl benzoate component includes a C12 alkyl benzoate, a C13 alkyl benzoate, a C14 alkyl benzoate, a C15 alkyl benzoate, or a mixture thereof. Neopentyl glycol diheptanoate (an example of which is represented by CAS 68855-18-5) is generally understood to be a mixture of texture-enhancing ingredient neopentyl glycol and grape-derived fatty acid heptanoic acid.

According to various aspects of the present disclosure, the antiperspirant composition can include cyclopentasiloxane as a solvent. However, in some aspects, the antiperspirant composition is substantially free of cyclopentasiloxane. For example, the antiperspirant can include less than about 10 wt% cyclopentasiloxane, less than about 9 wt%, less than about 8 wt%, less than about 7 wt%, less than about 6 wt%, less than about 5 wt%, less than about 4 wt%, less than about 3 wt%, less than about 2 wt%, less than about 1 wt%, less than about 0.5 wt%, less than about 0.1 wt%, less than about 0.01 wt%, or less than about 0.001 wt. in some aspects, the composition is completely free (e.g., includes 0 wt%) of cyclopentasiloxane. The disclosed isododecane component, alkyl benzoate component, and neopentyl glycol diheptanoate component, together, function to effectively replace cyclopentasiloxane in an antiperspirant while yielding an antiperspirant having similar, equal, or superior performance. Replacing cyclopentasiloxane can be desirable because, despite its performance in antiperspirant compositions, regulatory schemes seek to limit cyclopentasiloxane's use in cosmetic products. Therefore, it is important to find an effective substitute for cyclopentasiloxane. The inventors found that the combination of the isododecane component, alkyl benzoate component, and neopentyl glycol diheptanoate component, unexpectedly, provide an adequate replacement for cyclopentasiloxane. For example, an intensity or quantity at least one of spray discharge, amount of residue build-up in an underarm, amount of residue staining of clothing, perceived stickiness, antiperspirant protection, anti-odor protection, softness perception, or a combination thereof of the antiperspirant composition is substantially equal to that of the comparative antiperspirant composition including cyclopentasiloxane.

The antiperspirant composition can include any number of additional components. For example, the antiperspirant composition can include a perfume. The perfume can be in a range of from about 0.001 wt% to 8 wt% of the antiperspirant composition, 0.001 wt% to about 5 wt% of the antiperspirant composition, about 0.01 wt% to about 0.05 wt%, less than, equal to, or greater than about 0.001 wt%, 0.005, 0.01, 0.05, 0.1, 0.5, 1, 1.5, 2, 2.5, 3, 3.5, 4, 4.5, or about 5 wt%. Perfumes or perfume raw materials can include compounds having a thiol moiety can include 5-methyl-5-sulfanylhexan-3-one; 2-(4-methyl-1-cyclohex-3-enyl)propane-2-thiol; 5-methyl-2-(2-sulfanylpropan-2-yl)cyclohexan-1-one; 4,7,7-trimethyl-6-thiabicyclo[3.2.1]octane; 4-methoxy-2-methylbutane-2-thiol; methanethiol; ethanethiol; prop-2-ene-1-thiol; propane-2-thiol; 2-methylpropane-2-thiol; propane-1-thiol; butane-2-thiol; butane-1-thiol; 2-methylpropane-1-thiol; methyldisulfanylmethane; 2-methylbutane-2-thiol; 3-methylbutane-2-thiol; 3-methylbutane-2-thiol; pentane-2-thiol; pentane-1-thiol; 2-methylbutane-1-thiol; cyclopentanethiol; 3-methyldisulfanylprop-1-ene; methylsulfanyldisulfanylmethane; 1-methyldisulfanylpropane; ethane-1,2-dithiol; 1-(methyldisulfanyl)prop-1-ene; 3-sulfanylbutan-2-one; ethyldisulfanylethane; hexane-1-thiol; 1-ethyldisulfanylpropane; thiophene-2-thiol; propane-1,3-dithiol; 3-sulfanylpentan-2-one; 2-propan-2-yldisulfanylpropane; butane-1,4-dithiol; benzenethiol; ethylsulfanyldisulfanylethane; 3-methylsulfanyldisulfanylprop-1-ene; 1-methylsulfanyldisulfanylpropane; butane-2,3-dithiol; 4-methyl-4-sulfanylpentan-2-one; 3-prop-2-enyldisulfanylprop-1-ene; 1-methoxyhexane-3-thiol; ethyl 2-sulfanylpropanoate; 1-(prop-2-enyldisulfanyl)propane; 1-propyldisulfanylpropane; 1-(4-hydroxy-3-methoxyphenyl)ethanone butane-1,3-dithiol; 1-propyldisulfanylprop-1-ene; 2-methylbenzenethiol; thiophen-2-ylmethanethiol; 3-sulfanylbutan-2-ol; phenylmethanethiol pentane-1,5-dithiol; 2-ethylbenzenethiol; 3-prop-2-enylsulfanyldisulfanylprop-1-ene; methyldisulfanyldisulfanylmethane; 1-propylsulfanyldisulfanylpropane; 2,7,7-trimethylbicyclo[3.1.1]heptane-2-thiol; 2,6-dimethylbenzenethiol; 2-phenylethanethiol; hexane-1,6-dithiol; 2-(methyldisulfanylmethyl)furan; pyridin-2-ylmethanethiol; 2-methoxybenzenethiol; (7,7-dimethyl-2-bicyclo[3.1.1]heptanyl)methanethiol; methyldisulfanylbenzene; 1-butyldisulfanylbutane; (4-methoxyphenyl)methanethiol; 2-sulfanylpropanoic acid; ethyl 2-methyldisulfanylpropanoate; (2E)-3,7-dimethylocta-2,6-diene-1-thiol; 3,7-dimethylocta-2,6-diene-1-thiol; pyrazin-2-ylmethanethiol; methyldisulfanylmethylbenzene; 2-methyl-5-(1-sulfanylpropan-2-yl)cyclohexane-1-thiol; octane-1,8-dithiol; 2-pyrazin-2-ylethanethiol; naphthalene-2-thiol; 2-oxo-3-sulfanylpropanoic acid; 2-thiophen-2-yldisulfanylthiophene; cyclohexyldisulfanylcyclohexane; 2-(furan-2-ylmethyldisulfanylmethyl)furan; phenyldisulfanylbenzene; benzyldisulfanylmethylbenzene; 8-Hydroxy-5-quinolinesulfonic acid; bis(3-methylbutyl) 2-sulfanylbutanedioate; 2-aminoethanesulfonic acid; 2-phenyl-3H-benzimidazole-5-sulfonic acid; and 2-methyl-2-sulfanylpentan-1-ol. The compounds comprising sulfide moiety is selected from the group consisting of 1-butylsulfanylbutane; 2-methylsulfanylpyrazine; 2-methyl-3-methylsulfanylpyrazine; 2-(methylsulfanylmethyl)pyrazine; and mixtures thereof. Non-limiting examples of compounds having a thiazole moiety can include 2-(2-methylpropyl)-1,3-thiazole; 2-(4-methyl-1,3-thiazol-5-yl)ethanol; 4-methyl-2-propan-2-yl-1,3-thiazole; 1-(1,3-thiazol-2-yl)ethanone; 2,4,5-Trimethylthiazole; 2-isopropyl-4-methylthiazole; 4-vinyl-5-methylthiazole; 2,4-Dimethyl-5-acetylthiazole 1,3-thiazole; 4-methyl-1,3-thiazole; 2,4-dimethyl-1,3-thiazole; 4,5-dimethyl-1,3-thiazole; 2,5-dimethyl-1,3-thiazole; 5-ethenyl-4-methyl-1,3-thiazole; 2-ethyl-4-methyl-1,3-thiazole; 4-ethyl-2-methyl-1,3-thiazole; 2-propyl-1,3-thiazole; 2,4,5-trimethyl-1,3-thiazole; 2-ethyl-1,3-thiazole; 2-ethoxy-1,3-thiazole; 2-butan-2-yl-1,3-thiazole; 5-methoxy-2-methyl-1,3-thiazole; 2-ethyl-4,5-dimethyl-1,3-thiazole; 1,3-benzothiazole; 2,5-diethyl-4-methyl-1,3-thiazole; 1-(1,3-thiazol-2-yl)propan-1-one; 4,5-dimethyl-2-(2-methylpropyl)-1,3-thiazole; 2-methyl-1,3-benzothiazole; 1-(2,4-dimethyl-1,3-thiazol-5-yl)ethanone; and 4-methyl-2-propan-2-yl-1,3-thiazole.

Non-limiting examples of further perfumes include compounds having an oxathiane moiety, which can include (2R,4S)-2-methyl-4-propyl-1,3-oxathiane, 2-methyl-4-propyl-1,3-oxathiane, and 2-pentyl-4-propyl-1,3-oxathiane.

Non-limiting examples of further perfumes include compounds containing oxygen, sulfur, and nitrogen include 2-(4-methyl-1,3-thiazol-5-yl)ethanol; 1-(1,3-thiazol-2-yl)ethanone; 6-methyl-7-Oxa-1-thia-4-azaspiro[4.4]nonane; 2-[(furan-2-ylmethyl)sulfanyl] - 5 -methylpyrazine; 2,4-Dimethyl-5-acetylthiazole; 2-ethoxy-1,3-thiazole; 5-methoxy-2-methyl-1,3-thiazole; 1-(4,5-dihydro-1,3-thiazol-2-yl)ethanone; 1-(1,3-thiazol-2-yl)propan-1-one; 1-(2,4-dimethyl-1,3-thiazol-5-yl)ethanone; 2-amino-4-methylsulfanylbutanoic acid; (2S)-2-amino-4-methylsulfanylbutanoic acid; 8-Hydroxy-5-quinolinesulfonic acid; 2-aminoethanesulfonic acid; 2-phenyl-3H-benzimidazole-5-sulfonic acid.

More specific examples of the thiol moiety can include a-methyl-5-sulfanylhexan-3-one; 2-(4-methyl-1-cyclohex-3-enyl)propane-2-thiol; 5-methyl-2-(2-sulfanylpropan-2-yl)cyclohexan-1-one; 4,7,7-trimethyl-6-thiabicyclo[3.2.1]octane; and 4-methoxy-2-methylbutane-2-thiol.

More specific examples of the sulfide moiety can include 1-butylsulfanylbutane; ethyl 3-methylsulfanylpropanoate; and 2-(methylsulfanylmethyl)furan.

More specific examples of compounds having a thiazole moiety can include 2-(2-methylpropyl)-1,3-thiazole; 2-(4-methyl-1,3-thiazol-5-yl)ethanol; 4-methyl-2-propan-2-yl-1,3-thiazole; 4-methyl-2-propan-2-yl-1,3-thiazole; and 1-(1,3-thiazol-2-yl)ethanone.

A more specific example of compounds having an oxathiane moiety can be (2R,4S)-2-methyl-4-propyl-1,3-oxathiane.

More specific examples of a compound comprising oxygen, sulfur, and nitrogen can include 2-(4-methyl-1,3-thiazol-5-yl)ethanol, 1-(1,3-thiazol-2-yl)ethanone; and 6-methyl-7-Oxa-1-thia-4-azaspiro[4.4]nonane.

In another example, the perfume raw materials can include sulfide moieties or thiazole moieties. The sulfide moieties can include 1-butylsulfanylbutane, 4,7,7-trimethyl-6-thiabicyclo[3.2.1]octane, and 2-methyl-3-methylsulfanylpyrazine. The thiazole moieties can include 1-(1,3-thiazol-2-yl)ethanone.

In another example, the perfume raw materials can be added to a base perfume in a group. Suitable groups can include group (a): 1-butylsulfanylbutane; (2R, 4S)-2-methyl-4-propyl-1,3-oxathiane; and 4-methoxy-2-methylbutane-2-thiol; group (b): 2-(4-methyl-1,3-thiazol-5-yl)ethanol; 7-Oxa-1-thia-4-azaspiro[4.4]nonane; and 6-methyl-, 1-(1,3-thiazol-2-yl)ethanone; group (c): 2-(methylsulfanylmethyl)furan; ethyl 3-methylsulfanylpropanoate; and 1-butylsulfanylbutane; group (d): 5-methyl-5-sulfanylhexan-3-one; 5-methyl-2-(2-sulfanylpropan-2-yl)cyclohexan-1-one; and 2-(4-methyl-1-cyclohex-3-enyl)propane-2-thiol; group (e): 2-(2-methylpropyl)-1,3-thiazole; 2-(4-methyl-1,3-thiazol-5-yl)ethanol; and 4-methyl-2-propan-2-yl-1,3-thiazole; and group (f): (2R,4S)-2-methyl-4-propyl-1,3-oxathiane; 2-(4-methyl-1-cyclohex-3-enyl)propane-2-thiol; and (NE)-N-[(6E)-2,4,4,7-tetramethylnona-6,8-dien-3 -ylidene]hydroxylamine

Suitable perfume raw materials may be obtained from: Symrise GmbH, with offices located at Muhlenfeldstrasse 1, Holzminden, 37603, Germany; International Flavors & Fragrances Inc., a New York corporation having an address at 521 W 57th Street, New York, NY 10019; Givaudan Suisse SA a Swiss corporation having an address at 1214 Vernier, Switzerland; Firmenich Inc., with offices located at 250 Plainsboro Rd., Plainsboro Township, NJ 08536, United States; and Takasago International Corporation (USA), with offices located at 4 Volvo Drive, Rockleigh, NJ 07647, United States.

Antiperspirants compositions can also incorporate desirable scents through inclusion of perfumes and perfume raw materials in perfume delivery systems. Certain perfume delivery systems, methods of making certain perfume delivery systems, and the uses of such perfume delivery systems are disclosed in U.S. Pre-Grant Publication No. 2007/0275866 A1 . The perfumes and perfume raw materials previously disclosed can be used in such perfume delivery systems. Such perfume delivery systems include: polymer-assisted delivery (PAD), molecule-assisted delivery (MAD), fiber-assisted deliver (FAD), amine-assisted delivery (AAD), cyclodextrin delivery system (CD), starch encapsulated accord (SEA), inorganic carrier delivery system (ZIC), and Pro-Perfume (PP). Examples of these perfume delivery systems are further described below.

In some aspects, the antiperspirant composition can include an odor entrapper. Where present, the odor entrapper is present in a range of from about 0.01 wt% to about 5 wt% of the antiperspirant composition, about 0.02 wt% to about 4 wt%, less than, equal to, or greater than about 0.01 wt%, 0.02, 0.05, 0.1, 0.5, 1, 1.5, 2, 2.5, 3, 3.5, 4, 4.5, or about 5 wt%.

Examples of suitable odor entrappers include, for example, solubilized, water-soluble, uncomplexed cyclodextrin. As used herein, the term "cyclodextrin" includes any of the known cyclodextrins such as unsubstituted cyclodextrins containing from six to twelve glucose units, especially, alpha-cyclodextrin, beta-cyclodextrin, gamma-cyclodextrin and/or their derivatives and/or mixtures thereof. The alpha-cyclodextrin includes six glucose units, the beta-cyclodextrin includes seven glucose units, and the gamma-cyclodextrin consists of eight glucose units arranged in a donut-shaped ring. The specific coupling and conformation of the glucose units give the cyclodextrins a rigid, conical molecular structure with a hollow interior of a specific volume. Cyclodextrin molecules are described in U.S. Patent No. 5,714,137 , and U.S. Patent No. 5,942,217. Suitable levels of cyclodextrin are from about 0.1% to about 5%, alternatively from about 0.2% to about 4%, alternatively from about 0.3% to about 3%, alternatively from about 0.4% to about 2%, by weight of the composition.

In some aspects, the antiperspirant composition can further include a buffering agent. Where present, the buffering agent can be in a range of from about 0.001 wt% to about 0.75 wt% of the antiperspirant composition, about 0.01 wt% to about 0.5 wt%, less than, equal to, or greater than about 0.001 wt%, 0.005, 0.01, 0.05, 0.1, 0.5, or about 0.75 wt%.

A buffering agent can be alkaline, acidic or neutral. The buffer can be used in the composition for maintaining the desired pH. The composition may have a pH from about 3 to about 10, from about 4 to about 9, from about 5 to about 8, from about 6 to about 7, or it may have a pH of about 6.5. One unique feature of the polyvinyl amine malodor control polymers is its ability to maintain active nitrogen sites at high pH levels which can help enhance the antibacterial effect which comes, at least in part, from the nitrogen sites. Suitable buffering agents include, for example, hydrochloric acid, sodium hydroxide, potassium hydroxide, and combinations thereof.

In some aspects of the present disclosure, the antiperspirant composition can include a preservative. Where present, the preservative can be a range of from about 0.0001 wt% to about 1 wt%, about 0.0001 wt% to about 0.5 wt% of the antiperspirant composition, about 0.0003 wt% to about 0.1 wt%, less than, equal to, or greater than about 0.0001 wt%, 0.0003, 0.0005, 0.001, 0.005, 0.01, 0.05, 0.1, or about 0.5.

When included, the preservative is included in an amount sufficient to prevent spoilage or prevent growth of inadvertently added microorganisms for a specific period of time, but not sufficient enough to contribute to the odor neutralizing performance of the composition. In other words, the preservative is not being used as the antimicrobial compound to kill microorganisms on the surface onto which the composition is deposited in order to eliminate odors produced by microorganisms. Instead, it is being used to prevent spoilage of the composition in order to increase shelf-life.

The preservative can be any organic preservative material which will not cause damage to fabric appearance, e.g., discoloration, coloration, bleaching. Suitable water-soluble preservatives include organic sulfur compounds, halogenated compounds, cyclic organic nitrogen compounds, low molecular weight aldehydes, parabens, propane diol materials, isothiazolinones, quaternary compounds, benzoates, low molecular weight alcohols, dehydroacetic acid, phenyl and phenoxy compounds, or mixtures thereof.

Non-limiting examples of commercially available water-soluble preservatives include a mixture of about 77% 5-chloro-2-methyl-4-isothiazolin-3-one and about 23% 2-methyl-4-isothiazolin-3-one, a broad spectrum preservative available as a 1.5% aqueous solution under the trade name Kathon® CG by Rohm and Haas Co.; 5-bromo-5-nitro-1,3-dioxane, available under the tradename Bronidox L® from Henkel; 2-bromo-2-nitropropane-1,3-diol, available under the trade name Bronopol® from Inolex; 1,1'-hexamethylene bis(5-(p-chlorophenyl)biguanide), commonly known as chlorhexidine, and its salts, e.g., with acetic and digluconic acids; a 95:5 mixture of 1,3-bis(hydroxymethyl)-5,5-dimethyl-2,4-imidazolidinedione and 3-butyl-2-iodopropynyl carbamate, available under the trade name Glydant Plus® from Lonza; N-[1,3-bis(hydroxymethyl)2,5-dioxo-4-imidazolidinyl]-N,N'-bis(hydroxy-methyl) urea, commonly known as diazolidinyl urea, available under the trade name Germall® II from Sutton Laboratories, Inc.; N,N"-methylenebis {N'-[1-(hydroxymethyl)-2,5-dioxo-4-imidazolidinyl]urea}, commonly known as imidazolidinyl urea, available, e.g., under the trade name Abiol® from 3V-Sigma, Unicide U-13® from Induchem, Germall 115® from Sutton Laboratories, Inc.; polymethoxy bicyclic oxazolidine, available under the trade name Nuosept® C from Hüls America; formaldehyde; glutaraldehyde; polyaminopropyl biguanide, available under the trade name Cosmocil CQ® from ICI Americas, Inc., or under the trade name Mikrokill® from Brooks, Inc; dehydroacetic acid; and benzsiothiazolinone available under the trade name Koralone™ B-119 from Rohm and Hass Corporation.

The antiperspirant composition can further include an inactive component. The inactive component can be in a range of from about 50 wt% to about 90 wt% of the antiperspirant composition, about 75 wt% to about 85 wt% of the antiperspirant composition, less than, equal to, or greater than about 50 wt%, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, or about 90 wt%. when the inactive component is present, the active component is in a range of from about 10 wt% to about 50 wt% of the composition, about 15 wt% to about 25 wt% of the composition, less than, equal to, or greater than about 10 wt%, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or about 50 wt% of the antiperspirant composition.

The concentration of the inactive component relative to the active component can impact the antiperspirant's performance. For example, if the inactive component concentration is too large, less perspiration may be blocked or prevented. Additionally, if the antiperspirant composition is an aerosol spray, too much inactive component can lead to blockage of the spray device that the antiperspirant composition is disposed in.

The inactive component can include a propellant. The propellant can be in a range of from about 15 wt% to about 100 wt% of the inactive component, about 80 wt% to about 95 wt% of the active component, less than, equal to, or greater than about 15 wt%, 20, 25, 30, 35, 40, 45, 50, 55, 60, 65, 70, 75, 80, 85, 90, or about 100 wt% of the inactive component. Examples of suitable propellants include compressed air, nitrogen, inert gases, carbon dioxide, and mixtures thereof. Propellants may also include gaseous hydrocarbons like propane, n-butane, isobutene, cyclopropane, and mixtures thereof. Halogenated hydrocarbons like 1,1-difluoroethane may also be used as propellants. Some non-limiting examples of propellants include 1,1,1,2,2-pentafluoroethane, 1,1,1,2-tetrafluoroethane, 1,1,1,2,3,3,3-heptafluoropropane, trans-1,3,3,3-tetrafluoroprop-1-ene, dimethyl ether, dichlorodifluoromethane (propellant 12), 1,1-dichloro-1,1,2,2-tetrafluoroethane (propellant 114), 1-chloro-1,1-difluoro-2,2-trifluoroethane (propellant 115), 1-chloro-1,1-difluoroethylene (propellant 142B), 1,1-difluoroethane (propellant 152A), monochlorodifluoromethane, and mixtures thereof. Some other propellants suitable for use include, but are not limited to, A-46 (a mixture of isobutane, butane and propane), A-31 (isobutane), A-17 (n-butane), A-108 (propane), AP70 (a mixture of propane, isobutane and n-butane), AP40 (a mixture of propane, isobutene and n-butane), AP30 (a mixture of propane, isobutane and n-butane), and 152A (1,1 diflouroethane).

In some examples, the inactive component can include a suspending agent. The suspending agent can be in a range of from about 0.1 wt% to about 5 wt% of the inactive component, about 0.1 wt% to about 2 wt% of the inactive component, less than, equal to, or greater than about 0.1 wt%, 0.5, 1, 1.5, 2, 2.5, 3, 3.5, 4, 4.5, or about 5 wt%. Where present, the suspending agent can include tearalkonium hectorite, which is designed to impart rheological control and suspension and is a suitable thickener for compositions of the present disclosure. It is a highly efficient rheological additive for intermediate to high polarity systems such as cyclomethicones, esters, triglycerides, vegetable oils, alcohols and ketones.

According to various aspects, of the present disclosure the antiperspirant composition can include an antiperspirant concentrate. In some aspects, alcohol is a predominant component of the concentrates provided herein. Useful alcohols include $C_1$-$C_3$ alcohols, with the preferred alcohol being ethanol. In certain examples, the alcohol is employed at a concentration level of from at least about 40%, 50% or 55% to about 80%, by weight of the inactive component.

According to various embodiments of the present disclosure, the antiperspirant composition can be a component of an assembly. For example, if the antiperspirant composition is an aerosol spray, the assembly can include a spray container and the antiperspirant composition. In some further examples, if the antiperspirant composition is a stick, the assembly can include a container with the antiperspirant composition disposed therein.

**Examples**

Various embodiments of the present disclosure can be better understood by reference to the following Examples which are offered by way of illustration. The present disclosure is not limited to the Examples given herein.

The synergistic effect of including an aluminum salt (aluminum sesquichlorohydrate) or magnesium salt (magnesium carbonate); an extract (propanediol, glycerine, nymphaea coerulea flower extract, and nelumbo nucifera flower extract); and a film former (sodium polyacrylate) is shown in the examples below. Table 1 shows formula 1, which includes the aluminium salt, film former, and extract. Table 2 shows comparative formula 1, which does not include the extract or film former. Table 3 shows comparative formula 2, which does not include the extract.

Table 1: Formula 1

| Ingredient | Wt% of total antiperspirant composition |
|---|---|
| Aluminium Sesquichlorohydrate | 5-10 |
| C12-15 Alkyl Benzoate | 1-5 |
| Isododecane | 1-5 |
| Diisopropyl Adipate | 0.5-2 |
| Neopentyl Glycol Diheptanoate | 0.5-3 |
| Disteardimonium Hectorite | 0.5-3 |
| Propylene Carbonate | 0.1-1 |
| Sodium Polyacrylate | 0.5-2 |
| Aqua | 0.05-0.3 |
| Propanediol | 0.05-0.20 |
| Glycerine | 0.01-0.05 |
| Nymphaea Coerulea Flower Extract | 0.0005-0.009 |
| Nelumbo Nucifera Flower Extract | 0.0005-0.009 |
| Butane | 50-60 |
| Propane | 10-20 |
| Isobutane | 5-15 |

Table 2: Comparative Formula 1

| Ingredient | Wt% of total antiperspirant composition |
|---|---|
| Aluminium Sesquichlorohydrate | 5-10 |

(continued)

| Ingredient | Wt% of total antiperspirant composition |
|---|---|
| C12-15 Alkyl Benzoate | 3-8 |
| Isododecane | 1-5 |
| Diisopropyl Adipate | 1-5 |
| Neopentyl Glycol Diheptanoate | 0.5-2 |
| Disteardimonium Hectorite | 0.3-0.8 |
| Propylene Carbonate | 0.10-0.5 |
| Butyl Hydroxytoluene (BHT) | 0.005-0.01 |
| Butane | 50-60 |
| Propane | 10-20 |
| Isobutane | 5-15 |

Table 3: Comparative Formula 2

| INCI | % |
|---|---|
| Aluminium Sesquichlorohydrate | 3-12 |
| C12-15 Alkyl Benzoate | 1-5 |
| Isododecane | 1-5 |
| Diisopropyl Adipate | 0.5-2 |
| Neopentyl Glycol Diheptanoate | 0.5-2 |
| Disteardimonium Hectorite | 0.3-0.9 |
| Propylene Carbonate | 1-5 |
| Butyl Hydroxytoluene (BHT) | 0.01-0.8 |
| Sodium Polyacrylate | 0.5-3 |
| Aqua | 0.1-0.5 |
| glycerine | 0.05-1.5 |
| Humulus Lupulus Extract | 0.0001-0.0007 |
| Rosmarinus Officinalis Leaf Extract | 0.0001-0.0007 |
| Hamamelis Virginiana Leaf Extract | 0.0001-0.0007 |
| Salvia Officinalis Leaf Extract | 0.0001-0.0007 |
| Equisetum Arvense Extract | 0.0001-0.0007 |
| Citrus Limon Peel Extract | 0.0001-0.0007 |
| Centella Asiatica Extract | 0.0001-0.0007 |
| Pinus Sylvestris Bud Extract | 0.0001-0.0007 |
| Sodium Benzoate | 0.0001-0.0007 |
| Potassium Sorbate | 0.0001-0.0007 |
| Butane | 50-60 |
| Propane | 10-20 |
| Isobutane | 5-15 |

Table 4: Formula 2

| INCI Name | Wt% |
|---|---|
| Butane | 50-65 |
| Propane | 11-19 |
| Isobutane | 7-11 |
| C12-15 alkyl benzoate | 7-11 |
| Isododecane | 1-4 |
| Magnesium Carbonate | 0.5-1.5 |
| Diisopropyl Adipate | 0.5-1 |
| Neopentyl Glycol Diheptanoate | 0.2-0.9 |
| Disteardimonium Hectorite | 0.2-0.6 |
| Propylene Carbonate | 0.05-0.2 |
| Sweat Reduction of at least 20% in subjects after 12 hours, 24 hours, and 48 hours | 59.09% |

Table 5: Formula 3

| INCI Name | Wt% |
|---|---|
| Butane | 50-65 |
| Propane | 11-19 |
| Isobutane | 7-11 |
| C12-15 alkyl benzoate | 7-11 |
| Isododecane | 1-4 |
| Magnesium Carbonate | 0.5-1.5 |
| Diisopropyl Adipate | 0.5-1 |
| Neopentyl Glycol Diheptanoate | 0.2-0.9 |
| Disteardimonium Hectorite | 0.2-0.6 |
| Propylene Carbonate | 0.05-0.2 |
| Propanediol | 0.10-0.20 |
| Glycerin | 0.01-0.05 |
| Nymphaea Coerulea Flower Extract | 0.0005-0.003 |
| Nelumbo Nucifera Flower Extract | 0.0005-0.003 |
| Sweat Reduction of at least 20% in subjects after 12 hours, 24 hours, and 48 hours | 81.82% |

Table 6: Formula 4

| INCI Name | Wt% |
|---|---|
| Butane | 40-60 |
| Aqua | 15-25 |
| Propane | 10-15 |
| Isobutane | 5-10 |
| Isododecane | 2-7 |
| Neopentyl Glycol Diheptanoate | 0.5-3 |
| Dimethicone | 0.2-0.9 |
| PEG/PPG-18/18 Dimethicone | 0.05-0.3 |

(continued)

| INCI Name | Wt% |
|---|---|
| Propanediol | 0.05-0.2 |
| Sodium Benzoate | 0.05-0.2 |
| Triethyl Citrate | 0.05-0.2 |
| Caprylyl Glycol | 0.05-0.2 |
| Ethylhexylglycerin | 0.05-0.2 |
| Glycerin | 0.05-0.2 |
| Nymphaea Coerulea Flower Extract | 0.0005-0.002 |
| Nelumbo Nucifera Flower Extract | 0.0005-0.002 |
| Sweat Reduction of at least 20% in subjects after 12 hours, 24 hours, and 48 hours | 81.82% |

Table 7: Stick Formula 5

| INCI Name | Wt% |
|---|---|
| Propylene Glycol | 70-80 |
| Butylene Glycol | 5-15 |
| Sodium Stearate | 3-9 |
| Glycerin | 3-8 |
| Isosteareth-20 | 1-3 |
| Magnesium Salt | 0.5-1.5 |
| Steareth-2 | 0.3-0.9 |
| Ethylhexylglycerin | 0.1-0.5 |
| Propanediol | 0.05-0.20 |
| Water | 0.001-0.009 |
| Nymphaea Coerulea Flower Extract | 0.0005-0.005 |
| Nelumbo Nucifera Flower Extract | 0.0005-0.005 |
| Tocopherol | 0.00001-0.00030 |
| Sodium Hyaluronate | 0.00005-0.0006 |

Table 8: Roll-on Formula 6

| INCI | Wt % |
|---|---|
| Water | 70-90 |
| Helianthus Annuus (Sunflower) Seed Oil | 3-6 |
| Glycerin | 3-6 |
| Steareth-2 | 1-58 |
| Cetearyl Alcohol | 1-5 |
| Triethyl Citrate | 0.5-4 |
| Xanthan Gum | 0.5-4 |
| Steareth-20 | 0.5-1.5 |
| Ethylhexylglycerin | 0.1-1 |
| Sodium Benzoate | 0.1-1 |
| Chlorphenesin | 0.1-0.3 |

(continued)

| INCI | Wt % |
|---|---|
| Propanediol | 0.1-0.3 |
| Disodium EDTA | 0.05-2 |
| Tocopherol | 0.001-0.005 |
| Nymphaea Coerulea Flower Extract | 0.0005-0.003 |
| Nelumbo Nucifera Flower Extract | 0.0005-0.003 |
| Vegetable Oil | 0.0005-0.003 |
| Sodium Hyaluronate | 0.00005-0.0006 |

Table 9: Roll-on Formula 7

| INCI | % |
|---|---|
| Water | 60-80 |
| Helianthus Annuus (Sunflower) Seed Oil | 2-6 |
| Glycerin | 2-6 |
| Steareth-2 | 1-5 |
| Cetearyl Alcohol | 0.5-5 |
| Triethyl Citrate | 0.5-3 |
| Magnesium Salt | 0.5-3 |
| Xanthan Gum | 0.5-0.9 |
| Steareth-20 | 0.5-1.5 |
| Ethylhexylglycerin | 0.3-1 |
| Sodium Benzoate | 0.3-1<br>0.3-1 |
| Chlorphenesin | 0.1-5 |
| Propanediol | 0.05-3 |
| Disodium EDTA | 0.05-2.5 |
| Tocopherol | 0.0005-0.004 |
| Nymphaea Coerulea Flower Extract | 0.0005-0.005 |
| Nelumbo Nucifera Flower Extract | 0.0005-0.005 |
| Vegetable Oil | 0.0005-0.003 |
| Sodium Hyaluronate | 0.00005-0.0005 |

Table 10: Stick Formula 8

| INCI | % |
|---|---|
| Propylene glycol | 60-80 |
| Butylne glycol | 5-15 |
| Sodium stearate | 3-8 |
| Glycerin | 3-8 |
| Isosteareth-20 | 1-4 |
| Steareth-2 | 0.2-0.6 |
| Ethylhexylglycerin | 0.1-0.3 |

(continued)

| INCI | % |
|---|---|
| Propanediol | 0.05-0.15 |
| Water | 0.001-0.008 |
| Nymphaea Coerulea Flower Extract | 0.0005-0.005 |
| Nelumbo Nucifera Flower Extract | 0.0005-0.005 |
| Vegetable Oil | 0.0005-0.003 |
| Sodium Hyaluronate | 0.00005-0.0005 |

To study the amount of perspiration prevention provided by formulas 1-4 and comparative formulas 1 and 2, study subjects were assessed by a dermatologist at the start of the study in order to verify the inclusion and non-inclusion criteria. If included, the subjects received the products to be used during the wash-out period, and the instructions to be followed during this period. The wash-out period lasted 21 ± 4 days. After the washout period, subjects returned to the Institute. Subjects were exposed to heat (hot room sitting) for 80 minutes (Baseline). The temperature was 37.8°C ± 1°C and relative humidity between 30% and 40%. The subjects approved washed their armpits and the investigational product was applied. These procedures were repeated for three days, in which one product application was performed by day for three consecutive days. At this time-point, subjects received T-shirts for using during these three days. Ninety-six hours (after the third application of the investigational product (96h)), the subjects also undergone exposure to heat (Hot Room sitting) for perspiration collection evaluation under the same condition of Baseline.

In another example the ability of the antiperspirant of formula 1 was assessed for its ability to reduce sweat by 3X for a time of 96 hours. The results of formula 1 were compared to an untreated armpit, which was considered to be a control. The test was conducted in accordance with the protocol laid out in 21 CFR 350.60 of the final rule for OTC antiperspirant drug products, published in the Federal Register on June 9, 2003 (68 FR 34273).

The methodology consisted of suspending the use of any deodorant, ointment, cream or any other product in the armpits region for 21 days and sanitizing them only with neutral soap and a standard deodorant that was delivered by the researcher. After the conditioning period, the armpits were cleaned with a cleaning agent (neutral soap) and the initial evaluation was carried out in the sauna to verify the amount of sweat produced by the armpit without the application of any products. For approval, it was necessary for the participant to produce at least 100mg of armpit sweating for two consecutive sessions (T1 and T2) of 20 minutes each. Subsequently, the armpits were again cleaned with a neutral soap for the application of the investigational product in one armpit, while the other armpit remained without the application of any products. Four applications of the investigational product were carried out. After the application of the investigational product, the research participant was instructed not to wet, wash or rub any product in the armpits. 96 hours after the last application of the investigational product, the participants were exposed to heat in a sauna.

*Conditioning Phase*

During the conditioning period, participants were instructed to suspend the use of any topical products in the armpits. Participants were instructed to clean their armpits only with the neutral soap and standard deodorant provided by the laboratory. The conditioning period was 21 days.

*Verification of aluminum residue*

Participants were instructed to remove their T-shirt and lie on the stretcher with their arms up.
The researcher passed cotton-tipped flexible rod across the entire armpit region and then stored it in a properly identified plastic cup. Then, using a disposable pipette, applied the aluminum accusing solution until the tip of the cotton is well watered. If there is aluminum, the added solution will change its color for pink or purple.
All participants were approved in the verification of aluminum residue.

*Underarm hygiene*

Participants were instructed and supervised by the researcher to wash their armpits before applying the investigational product.
The armpits were cleaned according to the following procedure, before the baseline sauna and the first application of the investigational product:

- Wash one of the armpits during the period of 20 seconds with a standard neutral soap;
- Rinse the armpit under running water until all the soap is completely removed;
- Dry the armpit with a disposable towel;
- Repeat the same procedure with the other armpit.

After cleaning the armpits, the participants received and wore a white tank top, 100% cotton, cleaned with water and mild soap.

The armpits were cleaned according to the following procedure, before other applications of the investigational product:

- Wash one armpit for 10 seconds with a disposable towel saturated with a standard 2% neutral soap solution;
- Wet a clean disposable towel in running water and rinse the armpit until all soap is completely removed;
- Gently dry the armpit with a disposable towel; Repeat the same procedure with the other armpit.

*Initial assessment of armpits sweating*

Research participants remained in the sauna with a temperature of $37.8 \pm 2°C$ and relative air humidity = $35 \pm 5\%$, for a total period of 80 minutes, including 40 minutes of heating.

To collect sweating were used of absorbents pads, stored in polyethylene containers closed.

The pads were placed in the armpits of the participants immediately after they entered the sauna being replaced every 20 minutes. Every 20 minutes of warm up period, the pads were removed and discarded. Then, new pre-weighed pad was placed, being replaced every 20 minutes. The pads relative to the times of 60 to 80 minutes of exposure were removed and weighed, corresponding respectively to T1 and T2.

The participants drank 200 ml of water before entering the hot room, and then a further 200 ml of water after spending 40 minutes in the hot room.

In the initial assessment the research participants must present at least 100 mg of sweating in each armpit for 20 minutes of heat exposure.

*Application of the investigational product*

The investigational product was applied for 3 seconds timed, at a distance of 15 cm in a defined armpit area of 24 $cm^2$.

The procedure for applying the investigational product was performed four times by the researcher. The investigational product was evenly distributed in the armpits until it completely covered the defined area.

After the applications, the participants remained in an air-conditioned place for 30 minutes until the film was completely dried and formed.

*Assessment of underarm sweat after the application of the investigational product*

96 hours after the last application of the investigational product, the research participants were exposed to heat in the sauna.

The research participants remained in the sauna with a temperature of $37.8 \pm 2 °C$ and relative humidity = 30 to 40%, for a total period of 80 minutes.

For the collection of sweating, absorbent pads were used, stored in closed polyethylene packages. The first pads were placed on the participants' armpits immediately after they entered the sauna and they were changed every 20 minutes. After 40 minutes of warm-up, the pads were removed and discarded.

Then, new previously weighed pads were placed, being replaced every 20 minutes. The pads for the 60 and 80-minute exposure times were removed and weighed, corresponding to the T3 and T4 times, respectively.

The participants ingested 200 ml of water when entering the sauna and 40 minutes after its beginning, in order to maintain the internal level of hydration, allowing a free flow of sweat and evidencing only the effects of the formulation of the tested product. The levels obtained during the first 40 minutes were disregarded. Participants left the sauna, hydrated and waited 5 minutes for restoring and adapting to the outside temperature.

Research participants who had less than 100mg of sweat in the control armpit during 20 minutes of exposure to heat were excluded from the study.

*Results obtained in the assessment*

*Initial assessment of armpit sweating*

According to the data obtained, 22 research participants were approved in the initial phase of the study, that is, all had a quantity of sweating equal to or greater than 100mg per armpit at each time, T1 and T2.

*Assessment of armpit sweating after application of the investigational product*

When in the study there are pre-treatment records (baseline) the ratio of the treated armpit to the control armpit adjusted to the ratio of the right-to-left armpit sweating rate is defined for each individual by the formula:

$$Z = (PC \times T) / (PT \times C)$$

Where: Z is the adjusted ratio, PC is the amount of sweat obtained in the pre-treatment for the control armpit, PT is the amount of sweat obtained in the pre-treatment for the armpit that will receive the treatment, T is the amount of sweat obtained for the armpit treated with the investigational product and C is the amount of sweat obtained for the control armpit.

The test showed that at least 50% of the survey participants showed a reduction in sweating of at least 60% after 96 hours for Formula 1. The advantageous performance of Formula 1 is demonstrated as well against Comparative Formula 1 and Comparative Formula 2. Specifically, Comparative Formula 1, lacked comparable performance and lacks the NELUPURE composition. Comparative Formula 2 is a more traditional antiperspirant composition that includes several extracts other than NELUPURE. Formulas 2-4 show that magnesium salts such as magnesium carbonate can be used in antiperspirant compositions and deliver adequate performance. Formula 2 shows that magnesium carbonate can be used in an antiperspirant composition that does not include NELUPURE. Formula 3 shows that magnesium carbonate can be used in an anhydrous composition, including NELUPURE, and deliver adequate performance. Formula 4 shows that magnesium carbonate can be used in an aqueous composition, including NELUPURE, and deliver adequate performance. Formulas 5-8 show that it is possible to incorporate NELUPURE along with an aluminum or magnesium salt into a roll-on or stick antiperspirant composition.

**Claims**

1. An antiperspirant composition comprising:

   an active component comprising:

   > an aluminum salt, a magnesium salt or a mixture thereof;
   > a film former; and
   > an extract solution comprising:

   >> nymphaea coerulea flower extract; and,
   >> nelumbo nucifera flower extract,

   wherein the film former comprises Sodium Polyacrylate.

2. The antiperspirant composition of Claim 1, wherein the aluminum salt, magnesium salt or a mixture thereof is in a range of from 1 wt% to 55 wt%, preferably in a range of from 15 wt% to 45 wt% of the active component.

3. The antiperspirant composition of any one of Claims 1-2, wherein the aluminum salt comprises:

   $Al_2(OH)xQyXH_2O$,

   wherein
   Q is chlorine, bromine or iodine; x is from 2 to 5, and x+y= 6, and x and y do not need to be integers; and where X is from 1 to 6, preferably wherein the aluminum salt comprises aluminum sesquichlorohydrate.

4. The antiperspirant composition of any one of Claims 1-3, wherein the film former is in a range of from 1 wt% to 20 wt%, preferably in a range of from 7 wt% to 13 wt% of the active component.

5. The antiperspirant composition of any one of Claims 1-4, wherein the extract solution is in a range of from 0.10 wt% to 5 wt%, preferably from 0.1 wt% to 2 wt% of the active component.

6. The antiperspirant composition of any one of Claims 1-5, wherein the extract further comprises propanediol and glycerin.

7. The antiperspirant composition of Claim 6, wherein:

the nymphaea coerulea flower extract is in a range of from 0.00075 to 0.04 wt% of the extract;
the nelumbo nucifera flower extract is in a range of from 0.00075 to 0.04 wt% of the extract;
the propanediol is in a range of from 0.08 to 4 wt% of the extract; and
the glycerin is in a range of from 0.01 wt% to 1 wt% of the extract.

8. The antiperspirant composition of any one of Claims 1-7, further comprising a solvent, preferably wherein the solvent comprises a C12-C15 alkyl benzoate, isododecane, or a mixture thereof.

9. The antiperspirant composition of Claim 8, wherein the C12-C15 alkyl benzoate component comprises a C12 alkyl benzoate, a C13 alkyl benzoate, a C14 alkyl benzoate, a C15 alkyl benzoate, or a mixture thereof.

10. The antiperspirant composition of any one of Claims 1-9, wherein the antiperspirant composition is a stick antiperspirant, a body spray, a clear gel, or an aerosol antiperspirant.

11. The antiperspirant composition of any one of Claims 1-10, further comprising a perfume.

12. The antiperspirant composition of any one of Claims 1-11, further comprising a preservative.

13. The antiperspirant composition of any one of Claims 1-12, further comprising a propellant, preferably wherein the propellant is present in a range of from 15 wt% to 100 wt%, more preferably from 80 wt% to 95 wt% of the active component.

14. The antiperspirant composition of any one of claims 1-13, wherein the magnesium salt comprises magnesium carbonate or magnesium chloride.

15. An assembly comprising:

a spray or a roll-on container; and
the antiperspirant composition of any one of Claims 1-14.


**Patentansprüche**

1. Schweißhemmende Zusammensetzung, umfassend:
eine aktive Komponente, umfassend:

ein Aluminiumsalz, ein Magnesiumsalz oder eine Mischung davon;
einen Filmbildner; und
eine Extraktlösung, umfassend:

Nymphaea-coerulea-Blütenextrakt; und
Nelumbo-nucifera-Blütenextrakt,
wobei der Filmbildner Natriumpolyacrylat umfasst.

2. Schweißhemmende Zusammensetzung nach Anspruch 1, wobei das Aluminiumsalz, das Magnesiumsalz oder eine Mischung davon in einem Bereich von 1 Gew.-% bis 55 Gew.-%, vorzugsweise in einem Bereich von 15 Gew.-% bis 45 Gew.-%, der aktiven Komponente vorliegt.

3. Schweißhemmende Zusammensetzung nach einem der Ansprüche 1 bis 2, wobei das Aluminiumsalz umfasst:

$$Al_2(OH)xQyXH_2O,$$

wobei

Q Chlor, Brom oder Iod ist; x 2 bis 5 beträgt und x + y = 6, und wobei x und y keine ganzen Zahlen sein müssen; und wobei X 1 bis 6 beträgt, wobei das Aluminiumsalz vorzugsweise Aluminium-Sesquichlorhydrat umfasst.

4. Schweißhemmende Zusammensetzung nach einem der Ansprüche 1 bis 3, wobei der Filmbildner in einem Bereich von 1 Gew.-% bis 20 Gew.-%, vorzugsweise in einem Bereich von 7 Gew.-% bis 13 Gew.-%, der aktiven Komponente vorliegt.

5. Schweißhemmende Zusammensetzung nach einem der Ansprüche 1 bis 4, wobei die Extraktlösung in einem Bereich von 0,10 Gew.-% bis 5 Gew.-%, vorzugsweise von 0,1 Gew.-% bis 2 Gew.-%, der aktiven Komponente vorliegt.

6. Schweißhemmende Zusammensetzung nach einem der Ansprüche 1 bis 5, wobei der Extrakt ferner Propandiol und Glycerin umfasst.

7. Schweißhemmende Zusammensetzung nach Anspruch 6, wobei:

der Nymphaea-coerulea-Blütenextrakt in einem Bereich von 0,00075 bis 0,04 Gew.-% des Extrakts vorliegt;
der Nelumbo-nucifera-Blütenextrakt in einem Bereich von 0,00075 bis 0,04 Gew.-% des Extrakts vorliegt;
das Propandiol in einem Bereich von 0,08 bis 4 Gew.-% des Extrakts vorliegt; und
das Glycerin in einem Bereich von 0,01 Gew.-% bis 1 Gew.-% des Extrakts vorliegt.

8. Schweißhemmende Zusammensetzung nach einem der Ansprüche 1 bis 7, die ferner ein Lösungsmittel umfasst, wobei das Lösungsmittel vorzugsweise ein C12-C15-Alkylbenzoat, Isododecan oder eine Mischung davon umfasst.

9. Schweißhemmende Zusammensetzung nach Anspruch 8, wobei die C12-C15-Alkylbenzoatkomponente ein C12-Alkylbenzoat, ein C13-Alkylbenzoat, ein C14-Alkylbenzoat, ein C15-Alkylbenzoat oder eine Mischung davon umfasst.

10. Schweißhemmende Zusammensetzung nach einem der Ansprüche 1 bis 9, wobei die schweißhemmende Zusammensetzung ein Antitranspirant in Stiftform, ein Körperspray, ein klares Gel oder ein Antitranspirant in Aerosolform ist.

11. Schweißhemmende Zusammensetzung nach einem der Ansprüche 1 bis 10, die ferner einen Duftstoff umfasst.

12. Schweißhemmende Zusammensetzung nach einem der Ansprüche 1 bis 11, die ferner ein Konservierungsmittel umfasst.

13. Schweißhemmende Zusammensetzung nach einem der Ansprüche 1 bis 12, die ferner ein Treibmittel umfasst, wobei das Treibmittel vorzugsweise in einem Bereich von 15 Gew.-% bis 100 Gew.-%, mehr bevorzugt von 80 Gew.-% bis 95 Gew.-%, der aktiven Komponente vorliegt.

14. Schweißhemmende Zusammensetzung nach einem der Ansprüche 1 bis 13, wobei das Magnesiumsalz Magnesiumcarbonat oder Magnesiumchlorid umfasst.

15. Anordnung, umfassend:

einen Sprüh- oder einen Roll-on-Behälter; und
die schweißhemmende Zusammensetzung nach einem der Ansprüche 1 bis 14.

**Revendications**

1. Composition antisudorale comprenant :
un composant actif comprenant :

un sel d'aluminium, un sel de magnésium ou un mélange de ceux-ci ;
un agent filmogène ; et

une solution d'extrait comprenant :

> un extrait de fleur de nymphaea coerulea ; et,
> un extrait de fleur de nelumbo nucifera,

dans laquelle l'agent filmogène comprend du polyacrylate de sodium.

2. Composition antisudorale selon la revendication 1, dans laquelle le sel d'aluminium, le sel de magnésium ou un mélange de ceux-ci se situe dans une plage de 1 % en poids à 55 % en poids, de préférence dans une plage de 15 % en poids à 45 % en poids du composant actif.

3. Composition antisudorale selon l'une quelconque des revendications 1 et 2, dans laquelle le sel d'aluminium comprend :

$$Al_2(OH)xQyXH_2O,$$

où
Q représente chlore, brome ou iode ; x vaut 2 à 5, et x + y = 6, et x et y ne sont pas nécessairement des nombres entiers ; et où X vaut 1 à 6, de préférence dans laquelle le sel d'aluminium comprend du sesquichlorohydrate d'aluminium.

4. Composition antisudorale selon l'une quelconque des revendications 1 3, dans laquelle l'agent filmogène se situe dans une plage de 1 % en poids à 20 % en poids, de préférence dans une plage de 7 % en poids à 13 % en poids du composant actif.

5. Composition antisudorale selon l'une quelconque des revendications 1 à 4, dans laquelle la solution d'extrait est dans une plage de 0,10 % en poids à 5 % en poids, de préférence de 0,1 % en poids à 2 % en poids du composant actif.

6. Composition antisudorale selon l'une quelconque des revendications 1 à 5, dans laquelle l'extrait comprend en outre du propanediol et de la glycérine.

7. Composition antisudorale selon la revendication 6, dans laquelle :

> l'extrait de fleur de nymphaea coerulea se situe dans une plage de 0,00075 à 0,04 % en poids de l'extrait ;
> l'extrait de fleur de nelumbo nucifera se situe dans une plage de 0,00075 à 0,04 % en poids de l'extrait ;
> le propanediol est dans une plage de 0,08 à 4 % en poids de l'extrait ; et
> la glycérine est dans une plage de 0,01 % en poids à 1 % en poids de l'extrait.

8. Composition antisudorale selon l'une quelconque des revendications 1 à 7, comprenant en outre un solvant, de préférence dans laquelle le solvant comprend un benzoate d'alkyle en C12-C15, de l'isododécane, ou un mélange de ceux-ci.

9. Composition antisudorale selon la revendication 8, dans laquelle le composant benzoate d'alkyle en C12-C15 comprend un benzoate d'alkyle en C12, un benzoate d'alkyle en C13, un benzoate d'alkyle en C14, un benzoate d'alkyle en C15, ou un mélange de ceux-ci.

10. Composition antisudorale selon l'une quelconque des revendications 1 à 9, dans laquelle la composition antisudorale est un antisudoral en bâtonnet, un spray corporel, un gel transparent, ou un antisudoral en aérosol.

11. Composition antisudorale selon l'une quelconque des revendications 1 à 10, comprenant en outre un parfum.

12. Composition antisudorale selon l'une quelconque des revendications 1 à 11, comprenant en outre un conservateur.

13. Composition antisudorale selon l'une quelconque des revendications 1 à 12, comprenant en outre un propulseur, de préférence dans laquelle le propulseur est présent dans une plage de 15 % en poids à 100 % en poids, de manière davantage préférée de 80 % en poids à 95 % en poids du composant actif.

14. Composition antisudorale selon l'une quelconque des revendications 1 à 13, dans laquelle le sel de magnésium comprend du carbonate de magnésium ou du chlorure de magnésium.

**15.** Ensemble comprenant :

un pulvérisateur ou un contenant à bille ; et
la composition antisudorale de l'une quelconque des revendications 1 à 14.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017070115 A1
- US 3887692 A, Gilman
- US 3904741 A, Jones and Rubino
- US 7897799 B
- US 20070275866 A1
- US 5714137 A
- US 5942217 A

**Non-patent literature cited in the description**

- *Federal Register*, 09 June 2003, vol. 68 (110)
- *CHEMICAL ABSTRACTS*, 31807-55-3
- *CHEMICAL ABSTRACTS*, 68411-27-8
- *CHEMICAL ABSTRACTS*, 68855-18-5
- *Federal Register*, 09 June 2003